## Europäisches Patentamt

## European Patent Office

## Office européen des brevets

(19)

(11) Publication number: **0 110 422**
**A2**

(12) # EUROPEAN PATENT APPLICATION

(21) Application number: **83112148.8**

(22) Date of filing: **02.12.83**

(51) Int. Cl.³: **C 12 P 13/20**
// **C12R1/19**

(30) Priority: **03.12.82 JP 212998/82**

(43) Date of publication of application: **13.06.84**
**Bulletin 84/24**

(84) Designated Contracting States: **DE FR GB**

(71) Applicant: **Tanabe Seiyaku Co., Ltd.,**
**No. 21 Dosho-machi 3-chome Higashi-ku, Osaka-shi**
**Osaka-fu (JP)**

(72) Inventor: **Chibata, Ichiro, No. 13-2, Fujishirodai 4-chome,**
**Suita-shi Osaka-fu (JP)**
Inventor: **Kisumi, Masahiko, No. 12-20,**
**Morikitacho 4-chome, Higashinada-ku Kobe-shi**
**Hyogo-ken (JP)**
Inventor: **Nishimura, Noriyuki, No. 5-21,**
**Uozaki-Nakamachi 3-chome, Higashinada-ku Kobe-shi**
**Hyogo-ken (JP)**

(74) Representative: **Hansen, Bernd, Dr.rer.nat. et al,**
**Hoffmann, Eitle & Partner Patentanwälte**
**Arabellastrasse 4, D-8000 München 81 (DE)**

(54) **Process for producing L-aspartic acid.**

(57) A process for producing L-aspartic acid which comprises contacting

(a) a culture of a mutant of the genus Escherichia having high aspartase activity which has derived from a parent strain having aspartase activity and can grow in a medium containing L-aspartic acid either as a sole nitrogen source or as sole carbon and nitrogen sources;

(b) microbial cells collected from the culture; or

(c) a processed material of the microbial cells, with fumaric acid and ammonia to produce L-aspartic acid and then collecting L-aspartic acid thus produced.

# PROCESS FOR PRODUCING L-ASPARTIC ACID

The present invention relates to a process for producing L-aspartic acid, more particularly, it relates to a process for producing L-aspartic acid by using a microorganism of the genus Escherichia which has high aspartase activity.

It has hitherto been known that Escherichia coli has aspartase activity, and a method of enzymatic production of L-aspartic acid from ammonium fumarate (or fumaric acid and an ammonium salt) by using such a microorganism has been also known in the prior art [Bull. Agr. Chem. Soc., 24, 296 (1960); Appl. Microbiol., 27, 886 (1974); and Japanese Patent Publication Nos. 12553/1979 and 18867/1982]. However, aspartase activity of Escherichia coli used in such a conventional method is not so high as it is sufficient for industrial production of L-aspartic acid. In addition, it has been known that, in the presence of a readily assimilable carbon source such as glucose, formation of an enzyme responsible for utilization of another carbon source in Escherichia coli having aspartase activity is repressed, i.e. the microorganism is subjected to so-called catabolite repression [Biochem. J., 36, 619 (1942)]. Therefore, when cultivation of Escherichia coli is carried out by addition of a readily assimilable carbon source such as glucose to a medium, aspartase activity of the microorganism is insufficiently high and, hence, the yield of L-aspartic acid is decreased.

Under these circumstances, in order to establish an industrially advantageous process for producing L-aspartic acid, the present inventors have intensively studied. As the result, the present inventors have succeeded in obtaining mutants of genus *Escherichia* which have very high aspartase activity in comparison with that of their parent strain and can be advantageously utilized in the production of L-aspartic acid in an industrial scale.

The main object of the present invention is to provide an industrially advantageous process for producing L-aspartic acid. This object as well as other objects and advantages of the present invention will become apparent to those skilled in the art from the following description.

According to the present invention, there is provided a process for producing L-aspartic acid which comprises contacting

(a) a culture of a mutant of the genus *Escherichia* having high aspartase activity which has derived from a parent strain having aspartase activity and can grow in a medium containing L-aspartic acid either as a sole nitrogen source or as sole carbon and nitrogen sources;

(b) microbial cells collected from the culture; or

(c) a processed material of the microbial cells, with fumaric acid and ammonia to produce L-aspartic acid and then collecting L-aspartic acid thus produced.

The mutant having high aspartase activity to be used in the present invention can be obtained by using a microorganism of the genus *Escherichia* having aspartase activity as a parent strain. After the mutagenesis of the parent strain, the treated microorganism is cultivated in a medium containing L-aspartic acid either as a sole nitrogen source or as sole carbon and nitrogen sources to isolate a strain which can grow faster than the parent strain in the medium to give the desired mutant.

The microorganism to be used as the parent strain is not limited to a specific one so far as it is a microorganism of the genus Escherichia having aspartase activity. A suitable example of the parent strain is Escherichia coli ATCC 11303.

The mutagenesis of the parent strain can be carried out according to a conventional method. For example, the treatment with N-methyl-N'-nitro-N-nitroso-guanidine or ultraviolet radiation can be employed.

The concentration of L-aspartic acid in a medium used for cultivation of the microorganism treated by the mutagenesis is preferably about 0.005 to 5 % regardless of using L-aspartic acid either as a sole nitrogen source or as sole carbon and nitrogen sources. When L-aspartic acid is used as a sole nitrogen source, glucose, sucrose or the like can be used as a carbon source. For example, when glucose is used, its concentration in the medium is preferably about 0.1 to 10 %. Further, in both cases of using L-aspartic acid as a sole nitrogen source and using it as sole carbon and nitrogen sources, various salts, trace elements and the like used for a conventional nutrient medium can be added to the medium.

The method for cultivating the microorganism

- 4 -    0110422

treated by the mutagenesis is not limited to a specific one and the cultivation can be carried out according to a conventional method.  For example, a so-called continuous cultivation method ["Saikin, Phage Jikken-ho" (the separate volume of Tampakushitsu, Kakusan, Koso), p 35 (1972)] can be suitably employed.  In such a method, the microorganism treated by the mutagenesis is inoculated in a flask containing a medium and the flask is incubated at 10 to 45°C, preferably at 28 to 37°C with continuously pouring the additional medium into the flask at a constant rate of addition (e.g., degree of dilution: 0.05 to 0.5/hr), while the equal volume of the resulting culture is removed from the flask to concentrate and separate microbial cells which can quickly grow under such conditions.  Then, the culture thus obtained is spread on plates containing a medium having the same composition as that of the medium used in the continuous cultivation but containing about 0.1 to 5 % of L-aspartic acid and cultivated at 10 to 45°C, preferably at 28 to 37°C for 1 to 3 days.  A large colony grown on the plates is isolated to give the desired mutant having high aspartase activity such as 0.65 to 14 $\mu$mole/min/mg protein as measured by the method disclosed hereinafter.

The representatives of the mutant having high aspartase activity thus obtained are deposited with Fermentation Research Institute (FRI), Agency of Industrial Science and Technology, Ibaragi-ken, Japan under Budapest Treaty on November 13, 1982.  They are _Escherichia coli_ EAPc-28 (FERM BP-388), _Escherichia coli_ EAPc-110 (FERM BP-389), and _Escherichia coli_ EAPc-130 (FERM BP-390).

Aspartase activities of these mutants are about 2.5 times as high as that of the parent strain or more.

The mutant of the present invention can be cultivated by using a conventional nutrient medium containing carbon sources (e.g., glucose, sucrose, glycerol, fumaric acid), nitrogen sources (e.g., ammonium fumarate, ammonium sulfate), organic nutrients (e.g., yeast extract, peptone, corn steep liquor, meat extract), inorganic salts (e.g., potassium dihydrogen phosphate, magnesium sulfate), and the like. The cultivation can be carried out according to a conventional method. For example, after adjusting pH of a medium to 5.0 to 9.0, the mutant is inoculated into the medium and cultivated at 10 to 45 °C, preferably, at 28 to 37°C for 12 to 96 hours under aerobic conditions. In the medium, L-aspartic acid can be used as a carbon source as well as a nitrogen source and the preferred amount thereof is about 0.1 to 5 %.

According to the present invention, L-aspartic acid is produced by contacting the culture obtained by cultivation of the mutant as described above; microbial cells collected from the culture; or a processed material of the microbial cells with the substrates, i.e. fumaric acid and ammonia to effect L-aspartic acid-producing enzymatic reaction.

The collection of the microbial cells from the culture can be carried out according to a conventional method such as filtration, centrifugation, and the like.

Examples of the processed material of the microbial cells are washed microbial cells prepared by washing the microbial cells with physiological saline solution, phosphate buffer solution, carbonate buffer solution and the like; dried microbial cells prepared by drying the microbial cells according to a conventional method such as lyophilization, acetone treatment and the like; ground microbial cells prepared by grinding the microbial cells with glass beads, alumina and the like; autolysates of the microbial cells prepared by toluene, chloroform and the like; sonicates of the microbial cells prepared by sonic oscillator; microbial cell extracts prepared by french press; and immobilized preparations obtained by immobilizing the microbial cells or the above other processed materials thereof according to a known method such as gel conjugation method, adsorption method or the like. Examples of the immobilized preparations are those obtained by immobilizing the microbial cells or the above other processed materials thereof on supports, carriers or bases such as polyacrylamide gel, sulfur-containing polysaccharide (e.g., carrageenan, furcellaran, etc.) gel, collagen gel, alginic acid gel, polyvinyl alcohol gel, agar gel and the like. When polyacrylamide gel is used, the immobilization can be carried out according to the method disclosed in Japanese Patent Publication No. 1831/1978. When sulfur-containing polysaccharide gel is used, the method disclosed in Japanese Patent Laid Open Publication No. 6483/1978 can be employed. Further, when collagen gel, alginic acid gel, polyvinyl alcohol gel, agar gel or the like is used, the immobilization can be carried out according to the methods disclosed in Japanese Patent Laid Open Publication Nos. 144780/1976, 30582/1974, 80285/1974 and 133484/1976.

Fumaric acid and ammonia to be used as the substrates can be introduced into a L-aspartic acid-producing enzymatic reaction system in various forms. For example, they can be introduced into the reaction system in the form of ammonium fumarate or as fumaric acid or its salt and an inorganic ammonium salt.

As the salt of fumaric acid, for example, sodium fumarate or potassium fumarate can be suitably used. As the inorganic ammonium salt, for example, ammonium chloride, ammonium sulfate, ammonium phosphate or a mixture thereof can be suitably used.

In case of using fumaric acid or its salt and the inorganic ammonium salt, it is preferable that the molar ratio of these two components are within the range between 1 : 1.5 to 1 : 2.

The enzymatic reaction can be carried out at such a wide temperature range as about 5 to 50°C but, in view of stability of the enzyme of the mutant, it is preferable that the enzymatic reaction is carried out at 20 to 45 °C. Further, it is preferable to carry out the enzymatic reaction at a pH range of 6 to 10.  Besides, when the enzymatic reaction is carried out, it is preferable to add a divalent metal ion such as calcium ion, magnesium ion, manganese ion, strontium ion or the like to the reaction system.  The amount of the divalent metal ion can be about 0.1 to 10 mM and thereby stability of the enzyme can be improved.

When the microbial cells are used, preferably, the reaction is carried out in batchwise and L-aspartic acid can be produced by suspending the microbial cells collected from the culture thereof in a solution of the above substrates and stirring the suspension.  When the immobilized preparation is used, the reaction can be carried out not only by a batch process but also by a continuous process using a column packed with the immobilized preparation since the immobilized preparation is insoluble in water.  For example, the immobilized preparation is packed in a column and a substrate solution is passed down through the column at a suitable flow rate to obtain effluent containing only L-aspartic acid.  When the reaction is carried out by a batch process, L-aspartic acid can be produced by suspending the immobilized preparation in a substrate solution and stirring the suspension.  In the latter case, the immobilized preparation can be reused by separating it from the reaction mixture according to a standard method such as filtration or centrifugation.  The progress of the above reaction is effected by the amount of microbial cells, temperature, reaction time, flow rate of the substrate (particularly, linear velocity) and the like.

0110422

Optimal reaction conditions which can attain 100 % progress of the reaction can be readily found, for example, by suitably adjusting the flow rate of a substrate solution passing down through a column according to the amount of an immobilized preparation, in case of a continuous process using a column, or by suitably adjusting a reaction time, in case of a batch process.

L-Aspartic acid thus produced and accumulated in the reaction mixture can be readily separated and purified according to known methods, for example, by combining a conventional method using an ion exchange resin with other known methods.

According to the process of the present invention, L-aspartic acid can be produced from fumaric acid and ammonia in a very high yield within a short period of time since the mutant used in the process of the present invention has extremely high aspartase activity in comparison with a microorganism of the genus _Escherichia_ used in a conventional process for producing L-aspartic acid. Further, with respect to the increase in aspartase activity of the mutant used in the present invention, although any specific mechanism has not yet been clarified, it is assumed that a gene thereof responsible for the production of aspartase is probably mutated. In addition, most of the mutants of genus _Escherichia_ used in the present invention have such a characteristic that their aspartase activities are not decreased even in the presence of a readily assimilable carbon source such as glucose since they are released from catabolite inhibition. Therefore, when the mutant used in the present invention is cultivated, high aspartase activity can be revealed regardless of the kinds of carbon sources to be used and thereby L-aspartic acid can be produced in a high yield.

The following experiments and examples further illustrate the present invention in detail but are not to be construed to limit the scope thereof.

In the experiments and examples, aspartase activity was determined by contacting 1 M ammonium fumarate solution containing 1 mM magnesium chloride (pH 8.7) with the microbial cells or the processed material thereof, incubating at 37°C for 15 to 60 minutes to effect the enzymatic reaction, and measuring L-aspartic acid in the resulting reaction mixture by bioassay using Leuconostoc mesenteroides P60 [J. Biol. Chem., 172, 15 (1948)].

Experiment 1

Escherichia coli ATCC 11303 was treated with N-methyl-N'-nitro-N-nitrosoguanidine according to a standard method and $5 \times 10^{10}$ microbial cells thus treated were inoculated into a spinner flask (volume 50 ml, manufactured by Belco Co., U.S.A.) containing a medium (50 ml) composed of glucose (3.0 %), sodium L-aspartate (0.015 %), potassium dihydrogen phosphate (0.3 %), dipotassium hydrogen phosphate (0.7 %) and magnesium sulfate heptahydrate (0.01 %). Then, a continuous cultivation was carried out at the degree of dilution of 0.1/hr at 30°C for 10 days. The resulting culture was diluted $10^5$ to $10^6$ times with physiological

- 11 -

0110422

saline and spread on plates containing a medium having the same composition as the above except that the concentration of sodium L-aspartate was 0.5 % and 1.5 % of agar was added. After cultivation at 30°C for 1 to 2 days, resulting large colonies were picked up to isolate the desired mutants, Escherichia coli EAPc-28 (FERM BP-388) and Escherichia coli EAPc-110 (FERM BP-389). Aspartase activities of these mutants and the parent strain were measured. The results are shown in Table 1.

Table 1

| Microorganisms | Aspartase activity ($\mu$mole/min/mg protein) | |
|---|---|---|
| | with Glucose (3 %) | without Glucose |
| E.coli EAPc-28 | 0.65 | 14.0 |
| E.coli EAPc-110 | 4.5 | 10.5 |
| E.coli ATCC 11302 | 0.22 | 4.35 |

Basal medium: potassium dihydrogen phosphate 0.3 %, dipotassium hydrogen phosphate 0.7 %, magnesium sulfate heptahydrate 0.01 %, ammonium sulfate 0.1 % and sodium L-aspartate 0.2 % (pH 7.0).

As is seen from Table 1, in comparison with the parent strain, ATCC 11303, the aspartase activity of EAPc-28 strain is increased about 3 times both in the presence of and in the absence of glucose. In view of this, it is recognized that EAPc-28 strain is a mutant resulted from mutation of a gene responsible for the production of aspartase rather than release of catabolite repression.

Further, in comparison with the parent strain, ATCC 11303, the aspartase activity of EPAc-110 strain is increased about 20 times in the presence of glucose and about 2.2 times in the absence of glucose. In view of this, it is recognized that EAPc-110 strain is a mutant resulted from both mutation of a gene responsible for the production of aspartase and release of catabolite repression.

Experiment 2

According to the same manner as described in Experiment 1, Escherichia coli ATCC 11303 treated with N-methyl-N'-nitro-N-nitrosoguanidine was inoculated in a medium composed of sodium L-aspartate (0.1 %), potassium dihydrogen phosphate (0.3 %), dipotassium hydrogen phosphate (0.7 %) and magnesium sulfate heptahydrate (0.01 %) and continuously cultivated for 7 days. The resulting culture was diluted as in Experiment 1 and spreaded on plates containing a medium having the same composition as the above except that concentration of sodium L-aspartate was 0.5 % and 1.5 % of agar was added. After cultivation at 30°C for 1 to 2 days, resulting large colonies were picked up to isolate the desired mutant, Escherichia coli EAPc-130 (FERM BP-390). Aspartase activities of this mutant and the parent strain were measured. The results are shown in Table 2.

Table 2

| Microorganisms | Aspartase activity (μmole/min/mg protein) | |
|---|---|---|
| | with Glucose (3 %) | without Glucose |
| E.coli EAPc-130 | 5.5 | 10.8 |
| E.coli ATCC 11302 | 0.22 | 4.35 |

Basal medium is the same as that used in Experiment 1.

As is seen from Table 2, in comparison with the parent strain, ATCC 11303, aspartase activity of EAPc-130 is increased 25 times in the presence of glucose and about 2.3 times in the absence of glucose. In view of this, it is recognized that Escherichia coli EAPc-130 strain is a mutant resulted from both mutation of a gene responsible for the production of aspartase and release of catabolite repression.

Example 1

(1) Escherichia coli EAPc-28 was inoculated in a medium (500 ml, pH 7.0) containing ammonium fumarate (3 %), potassium dihydrogen phosphate (0.2 %), magnesium sulfate heptahydrate (0.05 %), corn steep liquor (4 %) and yeast extract (2 %) and cultivated with shaking at 37°C for 24 hours. The resulting culture was centrifuged to collect microbial cells and the cells were suspended in physiological saline (16 ml). To the suspension was added 3.2 % aqueous solution of GENU GEL WG (carrageenan manufactured by Kopenhagen Pectin Factory Ltd.) (64 ml) previously warmed to 40°C and mixed in a water bath at 40°C. The resulting mixture was added dropwise to 1 M aqueous ammonium fumarate solution containing 1 mM magnesium chloride (pH 8.5) to give immobilized Escherichia coli (61 g, wet weight) having aspartase activity in globular gel (3 mm diameter). The aspartase activity per 1 g of the resulting immobilized cells was 860 $\mu$mole/hr.

(2) The immobilized Escherichia coli prepared in the above (1) (61 g) was packed in a jacketed column (4 cm x 8 cm) and incubated at 37°C for 48 hours to activate the immobilized cells (aspartase activity: 1,586,000 $\mu$mole/hr). After activation, 1 M ammonium fumarate solution containing 1 mM magnesium chloride (pH 8.5, 1,000 ml) was passed down through the column at 37°C at the flow rate of 50 ml/hr. The effluent was adjusted to pH 2.8 to precipitate crystals. The crystals were filtered off to give L-aspartic acid (122 g).

Example 2

(1) The microbial cells of Escherichia coli EAPc-28 (8 g) were suspended in physiological saline (5 ml). To the suspension was added 2.2 % aqueous solution of GENU GEL WG containing 1 % locust bean gum (80 ml) previously warmed to 40°C and stirred. The resulting mixture was gelled by cooling. Further, 2 % aqueous potassium chloride solution (25 ml) was slowly added and the mixture was allowed to stand for 30 minutes. The gel was shaped into cubes of 3mm in side length and washed with 2 % aqueous potassium chloride solution. The gel (89.3 g) thus obtained was dipped in cooled ethanol (100 ml) and added thereto glutaraldehyde in such an amount that the final concentration thereof was 0.49 %. The mixture was allowed to stand with ice-cooling for 15 minutes. Then, the gel was filtered off and washed with 2 % aqueous potassium chloride solution to obtain an immobilized Escherichica coli (85.6 g, wet weight, aspartase activity: 47,013 $\mu$moles/hr/g cells).

(2) The immobilized Escherichia coli obtained in the above (1) (11 g) was packed in a jacketed column (1.6 cm x 12 cm) and 1 M aqueous ammonium fumarate solution containing 1 mM magnesium chloride (pH 8.5) was continuously passed through the column at 37°C at the flow rate of 6 ml/hr day and night and the aspartase activity thereof was measured with time. As the result, even after 20 days, decrease of the activity was scarcely observed.

Example 3

The microbial cells of Escherichia coli EAPc-28 (8 g) was suspended in 0.05 M phosphate buffer (pH 8.5, 50 ml) and sonicated at 9 Kc for 15 minutes. Then, the suspension was centrifuged and the resulting supernatant (38 ml) was passed through a column packed with Duolite-A7 (a weak basic ion exchange resin manufactured by Diamond Shamrock Chemical Co. in U.S.A.) (60 ml) at room temperature at the flow rate of 30 ml/hr. Then, the column was treated with a solution containing 0.1 M phosphate buffer (pH 8.5, 300 ml) and 4 % glutaraldehyde (300 ml) and excess glutaraldehyde was thoroughly washed out to give an immobilized enzyme.

The immobilized enzyme was packed in a column (volume 50 ml) and 1 M ammonium fumarate solution containing 1 mM magnesium chloride (pH 8.5, 500 ml) was passed through the column at the flow rate of 25 ml/hr. The combined effluent was adjusted to pH 2.8 to give L-aspartic acid (61 g).

Example 4

According to the same manner as in Example 3, a supernatant was obtained by using Escherichia coli EAPc-28. The supernatant was subjected to ammonium sulfate fractionation (saturation degree: 30 to 50 %) and the resulting precipitate was dissolved in water (5 ml). The solution was dialyzed overnight against water and the resulting dialysate was taken as an enzyme solution (standard aspartase). Then, the enzyme solution (2 ml) and 3.2 % aqueous GENU GEL WG solution (12 ml) were mixed in a water bath at 37°C. The mixture was added dropwise to 2 % aqueous potassium chloride solution to give globular gel (about 3 mm diameter). The aspartase activity of the resulting immobilized aspartase was 31.74 $\mu$moles/hr/g.

Example 5

Escherichia coli EAPc-28 was inoculated in a medium (pH 7.0, 500 ml) containing ammonium fumarate (3 %), potassium dihydrogen phosphate (0.2 %), magnesium sulfate heptahydrate (0.05 %), corn steep liquor (4 %) and yeast extract (2 %) and cultivated at 37°C for 24 hours. The culture was adjusted to pH 8.5 by addition of aqueous ammonia and ammonium fumarate (65 g) and Triton X-100 (500 mg) were added thereto. The mixture was allowed to stand at 37°C for 12 hours to effect the enzymatic reaction. After completion of the reaction, the reaction mixture was filtered, concentrated and then adjusted to pH 2.8 to precipitate crystals. The crystals were filtered off to obtain crude crystals of L-aspartic acid. The crude crystals were recrystallized from water to obtain L-aspartic acid (46.1 g).

### Example 6

(1) Escherichia coli EAPc-110 obtained in Experiment 1 was inoculated in a medium (1 liter) containing sodium L-aspartate (1.0 %), potassium dihydrogen phosphate (0.3 %), dipotassium hydrogen phosphate (0.7 %) and magnesium sulfate heptahydrate (0.01 %) and cultivated with shaking at 30°C for 10 hours. After cultivation, the culture was centrifuged to collect microbial cells. The microbial cells were suspended in physiological saline. To the cell suspension (12 ml, containing wet cells 6 g) was added 3.2 % aqueous GENU GEL WG solution (48 ml) previously warmed to 37°C and the mixture was thoroughly stirred. The resulting mixture was added dropwise to 1 M aqueous ammonium fumarate containing 1 mM magnesium chloride (pH 8.5) to give an immobilized Escherichia coli having aspartase activity in globular gel (about 3 mm diameter, 59.5 g, wet weight). The aspartase activity of 1 g of the immobilized cells was 86.1 $\mu$moles/hr.

(2) The above-obtained immobilized Escherichia coli (59.5 g) was packed in a jacketed column (4 cm x 8 cm) and incubated at 37°C for 48 hours to activate the immobilized cells. After activation (aspartase activity: 162,000 μmoles/hr), 1 M ammonium fumarate solution containing 1 mM magnesium chloride (pH 8.5, 1,000 ml) was passed down through the column at 37°C at the flow rate of 50 ml/hr. The effluent was adjusted to pH 2.8 to precipitate crystals. The crystals were filtered off to give L-aspartic acid (120 g).

Example 7

(1) Escherichia coli EAPc-130 was inoculated in the same medium (1 liter) as used in Example 6 and, according to the same manner as in Example 3, there was obtained a immobilized Escherichia coli (60 g, wet weight). The aspartase activity per 1 g of the immobilized cells was 114.5 μmoles/hr.

(2) The immobilized Escherichia coli (60 g) obtained in the above (1) was packed in a jacketed column (4 cm x 8 cm) and incubated at 37°C for 48 hours to activate the immobilizec cells. After activation (aspartase activity: 206,100 μmoles/hr), 1 M ammonium fumarte solution containing 1 mM magnesium chloride (pH 8.5, 1,000 ml) was passed down through the column at 37°C at the flow rate cf 50 ml/hr. The effluent was adjusted to pH 2.8 to precipitate crystals. The crystals were filtered off to give L-aspartic acid (121 g).

CLAIMS:

1. A process for producing L-aspartic acid which comprises contacting

(a) a culture of a mutant of the genus _Escherichia_ having high aspartase activity which has derived from a parent strain having aspartase activity and can grow in a medium containing L-aspartic acid either as a sole nitrogen source or as sole carbon and nitrogen sources;

(b) microbial cells collected from the culture; or

(c) a processed material of the microbial cells, with fumaric acid and ammonia to produce L-aspartic acid and then collecting L-aspartic acid thus produced.

2. A process according to claim 1, wherein the mutant is _Escherichia coli_ having high aspartase activity and can grow in a medium containing L-aspartic acid either as a sole nitrogen source or as sole carbon and nitrogen sources.

3. A process according to claim 2, wherein the mutant is _Escherichia coli_ EAPc-28 (FERM BP-388).

4. A process according to claim 2, wherein the mutant is _Escherichia coli_ EAPc-110 (FERM BP-389).

5. A process according to claim 2, wherein the mutant is _Escherichia coli_ EAPc-130 (FERM BP-390).

6. A process according to claim 1, wherein the fumaric acid and ammonia is used in the form of ammonium fumarate.

7. A process according to claim 1, wherein the contact is effected at about 5 to 50°C at pH of 6 to 10.